# EUROPEAN PATENT SPECIFICATION

(11) **EP 2 043 662 B1**
(45) Date of publication and mention of the grant of the patent: **09.09.2015**
(21) Application number: 07870731.2
(22) Date of filing: 26.07.2007
(51) Int. Cl.: A61K 31/711, A61K 48/00, C12N 5/00, C12N 15/00

(54) **METHODS AND COMPOSITIONS FOR TREATING DISEASE**
VERFAHREN UND ZUSAMMENSETZUNGEN ZUR BEHANDLUNG VON KRANKHEITEN
METHODES ET COMPOSITIONS DE TRAITEMENT DE MALADIE

(30) Priority: 26.07.2006 US 820381 P; 09.02.2007 US 889095 P
(43) Date of publication of application: 08.04.2009
(73) Proprietor: Intrexon Corporation, Blacksburg, VA 26060 (US)
(72) Inventor: REED, Thomas D., Blacksburg, VA 24060 (US); BEECH, Robert P., Cincinnati, OH 45242 (US)
(74) Representative: Hart, Deborah Mary
(86) International application number: PCT/US2007/016747
(87) International publication number: WO 2008/073154

(56) References cited:
- WO-A-99/42593
- CA-A1- 2 436 196
- US-A1- 2002 150 557
- US-A1- 2003 224 521
- US-A1- 2005 220 765
- SETH P ET AL: "Adenovirus-mediated gene transfer to human breast tumor cells: An approach for cancer gene therapy and bone marrow purging" CANCER RESEARCH, AMERICAN ASSOCIATION FOR CANCER RESEARCH, BALTIMORE, MD., US, vol. 56, no. 6, 15 March 1996 (1996-03-15), pages 1346-1351, XP002112678 ISSN: 0008-5472
- HIRAI MANABU ET AL: "Ex vivo purging by adenoviral p53 gene therapy does not affect NOD-SCID repopulating activity of human CD34+ cells" CANCER GENE THERAPY, vol. 8, no. 12, December 2001 (2001-12), pages 936-947, XP002571320 ISSN: 0929-1903
- RUI HONGBING ET AL: "Cell-specific expression of the diphtheria toxin A-chain coding sequence induces cancer cell suicide" CHINESE MEDICAL JOURNAL (ENGLISH EDITION), vol. 115, no. 6, June 2002 (2002-06), pages 869-873, XP002571321 ISSN: 0366-6999
- MITANI ET AL.: 'Adenovirus As An Integrating Vector' CURR. GENE THERAPY vol. 2, 2002, pages 135 - 144, XP008101837

## Description

### Background of the Invention

### Field of the Invention

The present invention relates to ex-vivo methods of obtaining compositions comprising live non-diseased cells suitable for treating a disease by destroying diseased cells in a subject by causing the selective expression of a lethal polypeptide in cells that are expressing at least one disease marker gene.

### Background of the Invention

Cancer is a set of diseases resulting from uncontrolled cell growth, which causes intractable pain and death for more than 300,000 people per year in the United States alone. Oncogenes are genes that, generally speaking, promote cancer cell growth. The development of cancer is believed to depend on the activation of oncogenes and the coincident inactivation of growth suppressor genes (Park, M., "Oncogenes" in The Genetic Basis of Human Cancer (B. Vogelstein et al., eds.) pp. 205-228 (1998)). Oncogenes are mutated, dominant forms of cellular proto-oncogenes that stimulate cell proliferation, while tumor suppressor genes are recessive and normally inhibit cell proliferation.

Treatment of cancer patients with chemotherapeutic agents remains the primary method of treating systemic disease and there is a direct association between chemotherapeutic dose intensity and clinical response rate. Increasing doses of chemotherapy, however, have significant side effects including the widespread destruction of bone marrow hematopoietic progenitor cells with concomitant destruction of peripheral myeloid and lymphoid cellularity. Stem cell transplantation is often used in conjunction with high dose chemotherapy to facilitate the recovery of the hematopoietic system following chemotherapy.

Allogeneic stem cell transplantation, which is transplantation of stem cells from a donor other than the patient is often used. The allogeneic transplant protocol, however, carries a high mortality rate due primarily to graft-versus-host disease (GVD), wherein the transplanted cells attack the patient's own tissues.

Autologous stem cell transplantation is a protocol wherein the patient's stem cells are isolated prior to the high dose chemotherapy and subsequently reinfused. Autologous transplantation avoids complications associated with GVD, but may result in the reinfusion of tumor cells from within the stem cell product. Reinfusion of tumor cells is important because gene marking studies have demonstrated that reinfused tumor cells can directly contribute to disease relapse and a poor clinical outcome. For example, in the cases of lymphoma, leukemia, breast cancer and neuroblastoma, at least some of the contaminating tumor cells in a peripheral blood stem cells transplantation protocol have the capacity to grow clonogenically *in vitro* (Ross, et al., Blood 82:2605-2610 (1993)) as well in the patient.

To avoid reinfusing the cancer cells into the patient undergoing autologous stem cell transplantation, practitioners have attempted to "purge" bone marrow cells of their contaminating tumor cells. Various approaches for the *ex vivo* purging of tumor cell contaminating stem cell populations have been developed. For example, the use of monoclonal antibodies against membrane antigens with cytotoxic drugs, toxins, phototherapy, and biological modifiers or cytotoxic drugs can reduce tumor contamination by 1 to 3 orders of magnitude (Seiden, et al., J. Infusional Chemotherapy 6:17-22 (1996)). In another protocol, antibodies that are directed towards tumor cells are conjugated to radioisotopes have been used in an attempt to purge tumor cells. The use of cytotoxic drugs and/or radioactivity may not be specific as tumor cells and progenitor cells often display a similar phenotype of cell surface proteins and the use of such techniques may delay engraftment. The selection of CD34⁺ hematopoietic progenitor cells has also been used to reduce tumor cell reinfusion, although at a much lower purging efficacy. Again, these methods of CD34-based selection may not be specific enough as tumor cells may often display the CD34 antigen.

Thus, there is a need in the art for new methods that specifically remove diseased cells from a cell population that is targeted for autologous transplantation.

### Summary of the Invention

The present invention relates to ex-vivo methods of obtaining live non-diseased cells, said cells being suitable for treating a disease by destroying diseased cells in a subject. In one embodiment, the methods comprise determining the activity of at least one disease marker gene within a population of cells obtained from a subject. A polynucleotide molecule that encodes a selectable marker and a lethal polypeptide is then introduced into the cells, where the expression of the lethal polypeptide is controlled by the promoter of at least one of the disease marker genes previously identified. A lethal peptide is defined as a polypeptide that is itself lethal to the cells. After introduction of the polynucleotide, the cells are exposed to selection conditions to obtain cells comprising the polynucleotide and then the cells are treated with conditions to induce the expression of the lethal polypeptide to destroy the cells that are expressing the disease marker gene(s). After destruction of the diseased cells, the remaining live cells, which did not express the lethal polypeptide to an extent necessary to kill the cells, are separated from the dead cells. The live cells are suitable for restoration to the subject.

In one embodiment of the invention, the polynucleotide introduced into the cells is excised prior to restoring the cells to the subject. In another embodiment, the polynucleotide is not excised from the cells prior to restoring the cells to the subject. This enables destruction of the restored cells *in vivo* in the advent of a recurrence of the disease.

Another embodiment of the invention relates to methods of individualizing treatment of a subject in need of treatment for an abnormal condition, comprising determining the activity of at least one disease marker gene within a population of cells obtained from a subject, isolating at least one promoter of the disease marker gene, and generating a therapeutic polynucleotide by directly or indirectly linking the promoter to a polynucleotide encoding a polypeptide that is lethal to said cells and placing it in a vector further comprising a selectable marker. The therapeutic polynucleotide is introduced into the cells and the cells are exposed to selection conditions to obtain cells comprising the polynucleotide and then treated with conditions to induce expression of the lethal polypeptide, thereby destroying cells expressing the disease marker gene. The remaining live, non-diseased cells are then suitable for restoration restored to the subject.

### Brief Description of the Drawings

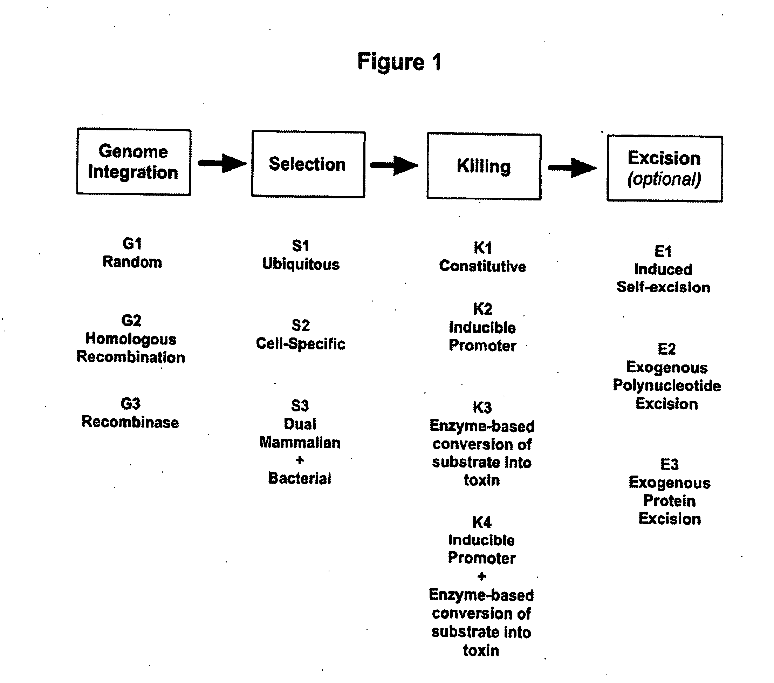

FIGURE 1 depicts a work flow diagram of a typical treatment process using the methods of the present invention. The methods depicted in Figure 1 comprise genomic integration, selection and killing. The constructs can optionally be excised from the genome. The figure also lists non-limiting variations of methods that are within the scope of the present invention. Any method of genomic integration, (Gl, G2 or G3) may be combined with any method of selection (S1, S2 or S3) and cell killing (Kl, K2, K3 or K4). In turn, any method of genomic excision (El, E2 or E3) may also be chosen, if the components that would allow excision are present in the genome.

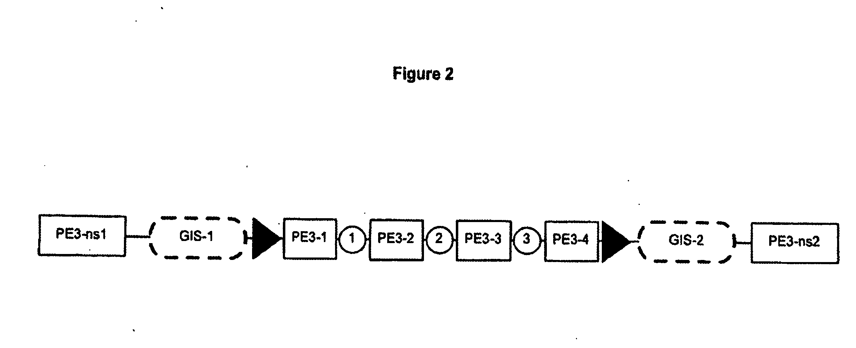

FIGURE 2 depicts one embodiment of the therapeutic polynucleotides of the present invention. In FIGURE 2, the PE3-1 gene program is a genome integration selector/reporter gene. The PE3-2 gene program is a disease marker gene promoter driving expression of lethal polypeptide, e.g., DTA. The PE3-3 gene program is an inducible promoter, *e.g.,* TetO, driving expression of a recombinase, *e.g.,* Cre. The PE3-4 gene program is a constitutive promoter driving expression of an inducer cDNA, *e.g.,* rTTA. The PE3-ns gene programs are negative selector/reporter genes that can be used to improve targeting efficiency. The arrowhead symbol represents *cis*-regulatory sequences, *e.g.,* loxP, that are recognized by a recombinase enzyme, e.g., Cre. Circles represent regions in the polynucleotide sequence that may comprise a chromatin modification domain (CMD). GIS-1 and GIS-2 are genomic integration sites.

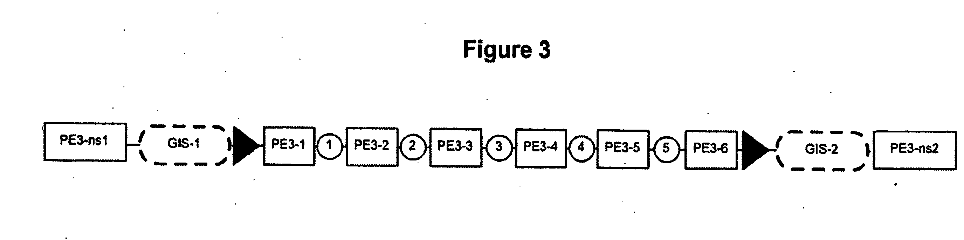

FIGURE 3 depicts another embodiment of the therapeutic polynucleotides of the present invention. In FIGURE 3, the PE3-1 gene program is a genome integration selector/reporter gene. The PE3-2 and PE3-3 gene programs are each a disease marker gene promoter driving expression of one of two halves of a Rheo transcription factor. The PE3-4 gene program is a Rheo promoter driving expression of a lethal polypeptide, e.g., DTA. The Rheo promoter requires the presence of the Rheo transcription factor, which is comprised of two subunits that bind together in the presence of ligand. Each of the subunits of the Rheo transcription factor is expressed in the PE3-2 and PE3-3 gene programs respectively. The PE3-5 gene program is a constitutive promoter driving expression of an inducer cDNA, *e.g.,* rTTA. The PE3-6 gene program is an inducible promoter, *e.g.,* TetO, driving expression of a recombinase, *e.g.,* Cre. The PE3-ns gene programs are negative selector/reporter genes that can be used to improve targeting efficiency. The arrowhead symbol represents *cis*-regulatory sequences, e.g., loxP, that are recognized by a recombinase enzyme, *e*.*g*., Cre. Circles represent regions in the polynucleotide sequence that may comprise a chromatin modification domain (CMD). GIS-1 and GIS-2 are genomic integration sites.

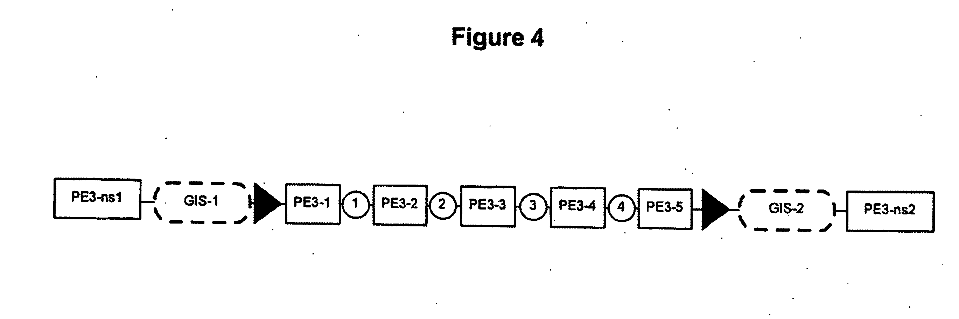

FIGURE 4 depicts another embodiment of the therapeutic polynucleotides of the present invention. In FIGURE 4, the PE3-1 gene program is a genome integration selector/reporter gene. The PE3-2 gene program is a disease marker gene promoter driving expression of lethal polypeptide, e.g., DTA. The PE3-3 gene program is a disease marker gene promoter driving expression of lethal polypeptide. The promoter and lethal polypeptide may be identical to or different from the promoter and lethal polypeptide in the PE3-2 gene program. The PE3-4 gene program is a constitutive promoter driving expression of an inducer cDNA, e.g., rTTA. The PE3-5 gene program is an inducible promoter, *e.g.,* TetO, driving expression of a recombinase, *e.g.,* Cre. The PE3-ns gene programs are negative selector/reporter genes that can be used to improve targeting efficiency. The arrowhead symbol represents *cis*-regulatory sequences, e.g., loxP, that are recognized by a recombinase enzyme, e.g., Cre. Circles represent regions in the polynucleotide sequence that may comprise a chromatin modification domain (CMD). GIS-1 and GIS-2 are genomic integration sites.

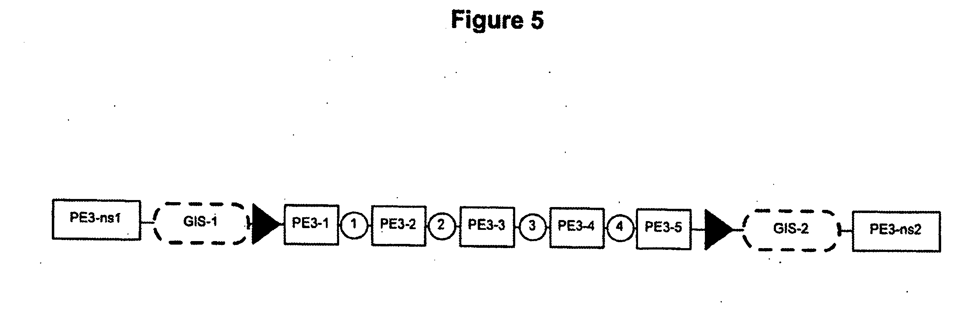

FIGURE 5 depicts one embodiment of the therapeutic polynucleotides of the present invention. In FIGURE 5, the PE3-1 gene program is a genome integration selector/reporter gene. The PE3-2 gene program is a disease marker gene promoter driving expression of lethal polypeptide, *e.g.,* DTA. The PE3-3 gene program is a constitutive promoter driving expression of an inducer cDNA, e.g., rTTA. The PE3-4 gene program is an inducible promoter, e.g., TetO, driving expression of a recombinase, *e.g.,* Cre. The PE3-5 gene program is a constitutive promoter driving expression of factors that promote de-differentiation of progenitor cells. The PE3-ns gene programs are negative selector/reporter genes that can be used to improve targeting efficiency. The arrowhead symbol represents *cis-*regulatory sequences, e.g., loxP, that are recognized by a recombinase enzyme, e.g., Cre. Circles represent regions in the polynucleotide sequence that may comprise a chromatin modification domain (CMD). GIS-1 and GIS-2 are genomic integration sites.

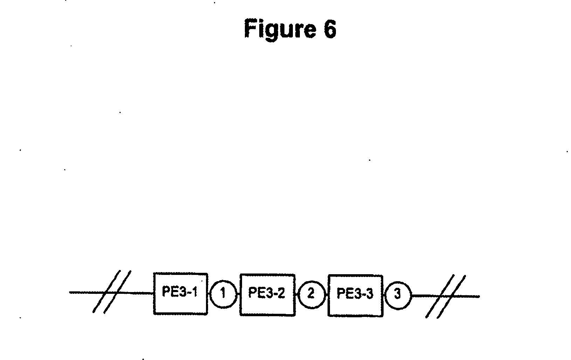

FIGURE 6 depicts one embodiment of the therapeutic polynucleotides of the present invention. In FIGURE 6, the PE3-1 gene program is the neo selector gene under the control of a constitutive promoter. The PE3-2 gene program is the disease marker gene promoter driving expression of an inducer cDNA, e.g., rTTA. The PE3-3 gene program is an inducible promoter, e.g., TetO, driving expression of the lethal polypeptide, *e.g.,* DTA. Circles represent regions in the polynucleotide sequence that can include the presence of a Chromatin Modification Domain (CMD).

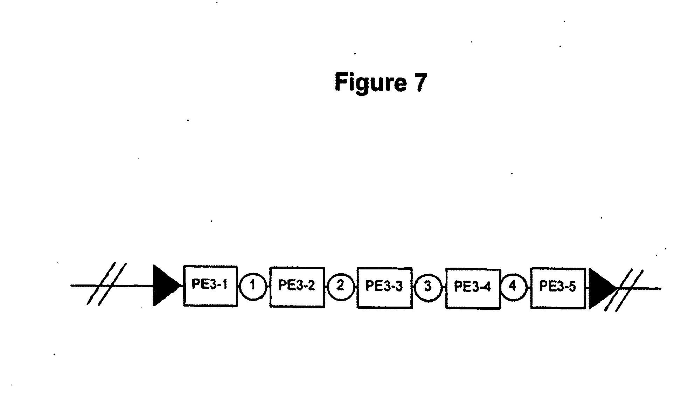

FIGURE 7 depicts one embodiment of the therapeutic polynucleotides of the present invention. In FIGURE 7, the PE3-1 gene program is a genome integration selector, e.g. neo, driven by a progenitor cell-specific promoter. The PE3-2 gene program is a disease marker gene promoter driving expression of an inducer cDNA, *e.g.,* rTTA. The PE3-3 gene program is an inducible promoter, *e*.*g*., TetO, driving expression of killer gene, e.g., DTA. The PE3-4 gene program is a constitutive promoter driving expression of second inducer cDNA, e.g., RheoCept^{®}. The PE3-5 gene program is an inducible promoter, *e.g.,* RheoSwitch^{®}, driving expression of a recombinase, *e.g.,* Cre, to delete the construct. Arrowhead symbols represent a *cis*-regulatory sequence recognized by a recombinase enzyme, e.g., loxP site. Circles represent a region in the polynucleotide sequence that can include the presence of a Chromatin Modification Domain (CMD).

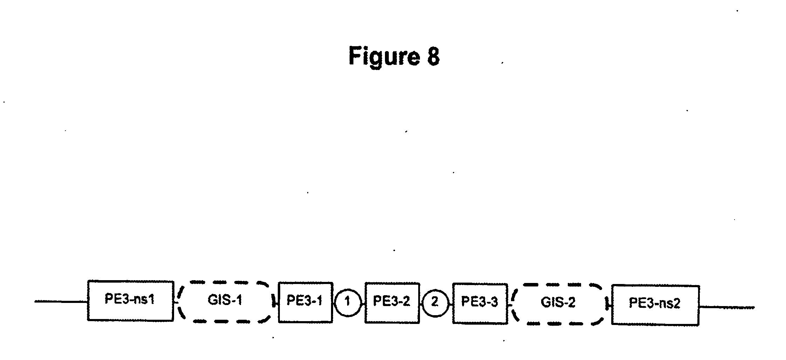

FIGURE 8 depicts one embodiment of the therapeutic polynucleotides of the present invention. In FIGURE 8, the PE3-1 gene program is a genome integration selector gene driven by a stem cell promoter. The PE3-2 gene program is a disease marker gene promoter driving expression of an inducer cDNA, e.g., rTTA. The PE3-3 gene program is an inducible promoter, *e.g.,* TetO, driving an enzyme-based substrate to lethal conversion of a killer gene, e.g., thymidine kinase. The PE3-ns gene programs are negative selector genes, e.g., a constitutive promoter driving expression of cytosine deaminase (CDA) or diphtheria (DTA) to improve targeting efficiency. Circles represent a region in the polynucleotide sequence that can include the presence of a Chromatin Modification Domain (CMD). GIS-1 and GIS-2 are genomic integration sites.

### Detailed Description of the Invention

The present invention relates to ex-vivo methods of obtaining live non-diseased cells suitable for treating a disease by destroying diseased cells in a subject. The methods comprise determining the activity of at least one disease marker gene within a population of cells obtained from a subject. A polynucleotide molecule that encodes a polypeptide that is lethal to the cells is then introduced into the cells, where the expression of the lethal polypeptide is controlled by the promoter of at least one of the disease marker genes previously identified. After introduction of the polynucleotide, the cells are treated with conditions to induce expression of the lethal polypeptide to destroy the cells that are expressing the disease marker gene(s). After destruction of the diseased cells, the remaining live cells, which did not express the lethal polypeptide to an extent necessary to kill the cells, are separated from the dead cells. The live cells are then suitable for restoration to the subject.

In one embodiment of the invention, the polynucleotide introduced into the cells is excised prior to restoring the cells to the subject. In another embodiment, the polynucleotide is not excised from the cells prior to restoring the cells to the subject. This enables destruction of the reintroduced cells *in vivo* in the advent of a recurrence of the disease.

As used herein, a "diseased cell" is used to mean a cell or cells that are abnormal in either their metabolism, histology, growth rate, mitotic rate or phenotype. As used herein, the term "phenotype," in relation to cells is used to mean the collection of proteins that a cell from a particular tissue or organ normally expresses. For example, the phenotypes of individual isolated cells can be assessed or classified based upon the presence or absence of cell surface markers such as clusters of differentiation (CD factors), which are cell surface antigens. While the phenotype of cells is generally considered to be the collection of proteins that the cell expresses or contains, it may only be necessary to determine the presence or absence of a single protein to adequately classify a cell into a given population or subpopulation or assess its phenotype. Thus, as used herein, the term "phenotype" is used to connote a particular population or subpopulation to which a cell belongs, based on the presence or absence of at least one protein or portion thereof. For example, particular embodiments of the present invention comprise isolating CD34⁺ cells that are normally found in peripheral blood and bone marrow. Continuing the example, the phenotype of a given population or subpopulation of isolated cells may simply be stated as CD34 positive (CD34⁺) or CD34 negative (CD34⁻). Of course, the methods of the present invention also contemplate determining the presence or absence of more than one protein to classify a cell into a given population or subpopulation of cells. Examples of CD proteins that may be used to classify cell phenotypes include, but are not limited to, CD3, CD38, CD59, CD49, CD54, CD61 (vitronectin receptor), CD71, CD73 (SH3), CD90 (Thy-1), CD105 (SH2), CD117, CD133, CD144 and CD166. Other proteins may also be used to determine the phenotype of a given cell or population of cells. Examples of other proteins that may be used to classify a cell's phenotype include, but are not limited to, transcription factors such as OCT4, cdx2, and Sox2, transporter proteins such as placental ABC transporter (ABC-p), and other cell surface antigens such as keratin sulphate-associated antigens, TRA-1-60, TRA-1-81, Thy-1 and the stage-specific embryonic antigens (SSEAs), *e.g.,* SSEA-1, SSEA-2, SSEA-3 and SSEA-4. Still more examples of proteins that may be used to classify a cell's phenotype include growth factor receptors such as the receptors for fibroblast growth factor (FGF), transforming growth factor-alpha (TGFa), transforming growth factor-beta (TGFβ), activin IIa, and bone morphogenic protein (BMP), as well as the major histocompatibility complex (MHC) proteins, *i.e.,* class I and class II MHC proteins. Still more examples of markers that may be used to identify cell phenotypes are CK (cytokeratin) 9, CK19, pdx-1, nestin, Pax-6, Nkx2.2, neurofilament, Tau, neuron-specific enolase (NSE), neurofilament protein (NF), microtubule associated protein 2 (MAP2), MAP2 kinase, glial fibrilliary acidic protein (GFAP) and cyclic nucleotide phosphodiesterase. In addition, it may also be possible to detect the presence or absence of portions or domains of proteins, and not the entire protein, to assess or classify a cell's phenotype. For example, some proteins may contain a src-homology domain (SH), such as SH1, SH2, SH3, SH4, etc., the presence or absence of which may be sufficient to adequately assess or classify a cell's phenotype, e.g., SH2⁺ or SH2⁻. As disclosed above, the phenotype of the cells may also be assessed or classified by the absence of particular proteins.

Methods of identifying cell phenotypes include, but are not limited to, standard immunohistochemistry techniques using antigen-specific antibodies, such as, for example, anti-CD34 antibodies. Other methods of assessing or classifying a cell's phenotype include, but are not limited to, standard blotting techniques such as Western blotting and Northern Blotting, and polymerase chain reaction (PCR) techniques, such as reverse transcriptase-PCR (RT-PCR). Indeed, it should be apparent that indirect methods, such as assays measuring or detecting mRNA, e.g., RT-PCR, can be used to assess or classify a cell's phenotype. Still other methods of assessing or classifying phenotypes of the cells include microarray techniques and flow cytometry techniques. Examples of flow cytometry techniques useful for sorting cells based upon their phenotype are disclosed in Practical Flow Cytometry, 3rd Edition, Wiley-Liss, Inc. (1995).

A diseased cell may, for example, have an abnormal phenotype as compared to other cells taken from the same source or tissue. For example, hematopoietic stem cells normally express CD34 and CD59, but do not express CD4, which is expressed normally by thymocytes, T helper cells, macrophages, Langerhans cells, dendritic cells or granulocytes. Any hematopoietic stem cell that expresses CD34, CD59 and CD4 may, for the purposes of the present invention, thus be considered to have an abnormal phenotype, *i.e.,* the cell is diseased. In one embodiment, the cells comprise hematopoietic stem cells, where at least a portion of the hematopoietic stem cells are diseased cells.

Other examples of stem cells include, but are not limited to, liver stem cells, mammary stem cells, pancreatic stem cells, neuronal stem cells, mesenchymal stem cells and non-human embryonic stem cells. The stem cells may or may not be pluripotent. "Pluripotent cells" include cells and their progeny, which may be able to differentiate into, or give rise to, pluripotent, multipotent, oligopotent and unipotent cells. "Multipotent cells" include cells and their progeny, which may be able to differentiate into, or give rise to, multipotent, oligopotent and unipotent progenitor cells, and/or one or more mature or partially mature cell types, except that the mature or partially mature cell types derived from multipotent cells are limited to cells of a particular tissue, organ or organ system. As used herein, "partially mature cells" are cells that exhibit at least one characteristic of the phenotype, such as morphology or protein expression, of a mature cell from the same organ or tissue. For example, a multipotent hematopoietic progenitor cell and/or its progeny possess the ability to differentiate into or give rise to one or more types of oligopotent cells, such as myeloid progenitor cells and lymphoid progenitor cells, and also give rise to other mature cellular components normally found in the blood. "Oligopotent cells" include cells and their progeny whose ability to differentiate into mature or partially mature cells is more restricted than multipotent cells. Oligopotent cells may, however, still possess the ability to differentiate into oligopotent and unipotent cells, and/or one or more mature or partially mature cell types of a given tissue, organ or organ system. One example of an oligopotent cell is a myeloid progenitor cell, which can ultimately give rise to mature or partially mature erythrocytes, platelets, basophils, eosinophils, neutrophils and monocytes. "Unipotent cells" include cells and their progeny that possess the ability to differentiate or give rise to other unipotent cells and/or one type of mature or partially mature cell type. As used herein, the term "progenitor cell" is used to mean cells and their progeny that can differentiate into at least partially mature cells, but lack the capacity for indefinite self-renewal in culture. Progenitor cells, as used herein, may be pluripotent, multipotent, oligopotent or even unipotent.

The methods of the present invention are carried out on a population of cells obtained from a subject in need of treatment. As used herein, the term "subject" is used interchangeably with the term "patient," and is used to mean an animal, in particular a mammal, and even more particularly a non-human or human primate.

As used herein, when used in reference to cells, the term "obtaining" is intended to mean any process of removing cells from a subject. The cells need not be isolated or purified when they are obtained from a subject. Cells may be obtained from any body fluid of a subject (e.g., blood, serum, urine, saliva, cerebral spinal fluid) or from tissue samples of a subject (e.g., biopsies, bone marrow aspirates). Once obtained, the desired cells may then be isolated.

As used herein, the term "isolated" or "isolating" or variants thereof, when used in reference to a cell or population of cells means that the cell or population of cells have been separated from a majority of the surrounding molecules and/or materials present which surround the cell or cells when the cell or cells were associated with a biological system *(e.g.,* bone marrow). The concentration of materials such as water, salts, and buffer are not considered when determining whether a cell has been "isolated." Thus, the term "isolated" is not intended to imply or indicate a purified population of cells of a particular phenotype, nor is it intended to mean a population of cells entirely devoid of debris, non-viable cells or cells of a different phenotype. The methods of isolating the cells should not limit the scope of the invention described herein. For example, the cells may be isolated using well-known methods, such as flow cytometry or other methods that exploit a cell's phenotype. Additional methods of isolating cells include using positive or negative selectors that the therapeutic constructs may contain, such that the cells may be isolated before or after introducing the therapeutic polynucleotide into the cells. Thus, in one embodiment, the construct may comprise a positive selector that can be used to "isolate" the desired cells from other cells that are initially obtained. As used herein, the term "purified," when used in reference to a cell or population of cells means that the cell or cells have been separated from substantially all materials which normally surround the cell or cells when the cell or cells were associated with a biological system. "Purified" is thus a relative term which is based on a change in conditions in terms of cells and/or materials in close proximity to the isolated cells being purified. Thus, isolated hematopoietic cells are considered to be purified even if at least some cellular debris, non-viable cells, cells of a different phenotype or cells or molecules such as proteins and/or carbohydrates are removed by washing or further processing, after the isolation. The term purified is not used to mean that the all of matter intended to be removed is removed from the cells being purified. Thus, some amount of contaminants may be present along with the purified cells.

In one embodiment, the activity of at least one disease marker gene is determined after the cells are obtained. In another embodiment, the activity of at least one disease marker gene is determined before the cells are obtained. Thus, the activity of one or more disease marker genes may be assumed or inferred if the disease or abnormal condition in the subject exhibits typical symptoms or markers of diseases or abnormal conditions where the activity of a set of disease marker genes has been established. As used herein, a disease marker gene is intended to mean a gene whose expression levels can be used to assess, diagnose or aid in the diagnosis of a disease or abnormal condition. Disease marker genes include genes that are expressed only in diseased cells and genes that are expressed in normal cells but are expressed at elevated levels in diseased cells. The most well-known examples of disease marker genes are oncogenes, but the methods of the present invention, however, are not limited to oncogenes. Examples of classes of oncogenes include, but are not limited to, growth factors, growth factor receptors, protein kinases, programmed cell death regulators and transcription factors. Specific examples of oncogenes include, but are not limited to, sis, erb B, erb B-2, ras, abl, myc and bcl-2 and TERT. Examples of other disease marker genes include tumor associated antigen genes and other genes that are overexpressed in diseased cells (e.g., MAGE-1, carcinoembryonic antigen, tyrosinase, prostate specific antigen, prostate specific membrane antigen, p53, MUC-1, MUC-2, MUC-4, HER-2/neu, T/Tn, MART-1, gp100, GM2, Tn, sTn, and Thompson-Friedenreich antigen (TF)).

Once the disease marker genes have been determined, promoters of these disease marker genes are placed into a therapeutic polynucleotide. As used herein, the term therapeutic polynucleotide is used to mean a polynucleotide that is introduced into the population of cells for the purpose of destroying the diseased cells. In one embodiment, the promoter is inserted into the polynucleotide such that it is operably linked to a portion of the polynucleotide that codes for a polypeptide that is lethal to the cells. The methods therefore exploit the abnormal activity of the diseased cell such that the diseased cell will ultimately destroy itself. As used herein, the term "operably linked" means a functional linkage between a nucleic acid expression control sequence (such as a promoter, or array of transcription factor binding sites) and a second nucleic acid sequence, wherein the expression control sequence directs transcription of the nucleic acid corresponding to the second sequence. In another embodiment, the promoter is indirectly linked to expression of the lethal polypeptide. For example, the disease marker promoter may be operably linked to a transcription factor that activates a second promoter that is operably linked to the polynucleotide encoding the lethal polypeptide.

The term "promoter" is used herein as it is in the art. Namely, the term promoter refers to a region of DNA that permits binding of an RNA polymerase to initiate transcription of a genetic sequence. The sequence of many disease marker genes, including the promoter region, is known in the art and available in public databases, e.g., GenBank. Thus, once a disease marker gene is identified in the obtained or isolated cells, the promoter sequence can be readily identified and obtained. Another aspect of the present invention is directed towards identifying a disease marker gene whose promoter can be isolated and placed into a therapeutic polynucleotide. The identity of the disease marker gene, therefore, may not be critical to specific embodiments of the present invention, provided the promoter can be isolated and used in subsequent settings or environments. The current invention thus includes the use of promoters from disease marker genes that are yet to be identified. Once new disease marker genes are identified, it can be a matter of routine skill or experimentation to determine the genetic sequences needed for promoter function. Indeed, several commercial protocols exist to aid in the determination of the promoter region of genes of interest. By way of example, Ding et al. recently elucidated the promoter sequence of the novel *Sprouty4* gene (Am. J. Physiol. Lung Cell. Mol. Physiol., 287: L52-L59 (2004)) by progressively deleting the 5'-flanking sequence of the human *Sprouty4* gene. Briefly, once the transcription initiation site was determined, PCR fragments were generated using common PCR primers to clone segments of the 5'-flanking segment in a unidirectional manner. The generated segments were cloned into a luciferase reporter vector and luciferase activity was measured to determine the promoter region of the human *Sprouty4* gene.

Another example of a protocol for acquiring and validating disease marker gene promoters includes the following steps: (1) acquire cancerous and non-cancerous cell/tissue samples of similar/same tissue type; (2) isolate total RNA or mRNA from the samples; (3) perform differential microarray analysis of cancerous and non-cancerous RNA; (4) identify candidate cancer-specific transcripts; (5) identify genomic sequences associated with the cancer-specific transcripts; (6) acquire or synthesize DNA sequence upstream and downstream of the predicted transcription start site of the cancer-specific transcript; (7) design and produce promoter reporter vectors using different lengths of DNA from step 6; and (8) test promoter reporter vectors in cancerous and non-cancerous cells/tissues, as well as in unrelated cells/tissues.

The source of the promoter that is inserted into the therapeutic polynucleotide can be natural or synthetic, and the source of the promoter should not limit the scope of the invention described herein. In other words, the promoter may be directly cloned from the obtained or isolated cells, or the promoter may have been previously cloned from a different source, or the promoter may have been synthesized.

In one embodiment, the promoter is operably linked to a polynucleotide that codes for a polypeptide that, when expressed, is lethal to the cell that expresses the polypeptide, because the polypeptide itself is lethal. As used herein, a polypeptide that is lethal to cells also includes polypeptides that induce cell death in any fashion, including but not limited to necrosis, apoptosis and cytotoxicity. Examples of polypeptides that can be lethal to cells include but are not limited to, apoptosis inducing tumor suppressor genes such as, but not limited to p53, Rb and BRCA-1, toxins such as diphtheria toxin (DTA), shigella neurotoxin, botulism toxin, tetanus toxin, cholera toxin, CSE-V2 and other variants of scorpion protein toxins to name a few, suicide genes such as cytosine deaminase and thymidine kinase, and cytotoxic genes, e.g., tumor necrosis factor, interferon-alpha. The present invention is not limited by the identity of the lethal protein, provided that the protein is capable of being lethal to the cell in which it is expressed.

In another embodiment, the disease marker gene promoter is indirectly linked to expression of the lethal polypeptide. In one embodiment, the disease marker gene promoter is operably linked to a polynucleotide that codes for a transcription factor and the polynucleotide coding for the lethal polypeptide is operably linked to a promoter that is activated by the transcription factor. The transcription factor may be a ligand-dependent transcription factor that activates transcription only in the presence of the ligand, e.g., members of the steroid receptor superfamily activated by their respective ligands *(e.g.,* glucocorticoid, estrogen, progestin, retinoid, ecdysone, and analogs and mimetics thereof) or rTTA activated by tetracycline. The transcription factor may be a naturally occurring polypeptide or a chimeric polypeptide comprising domains from two or more different transcription factors. For example, the ligand binding domain, transactivation domain, and DNA binding domain may each be obtained from two or three different transcription factors. In one embodiment, the transcription factor is one that is tightly regulated by the level of ligand that is present. In another embodiment, the domains of the transcription factor can be expressed on separate polypeptides such that activation of transcription occurs only when the two polypeptide dimerize together (and ligand is present). One example of such a system is the chimeric ecdysone receptor systems described in U.S. Patent No. 7,091,038, U.S. Published Patent Application Nos. 2002/0110861, 2002/0119521, 2004/0033600, 2004/0096942, 2005/0266457, and 2006/0100416, and International Published Application Nos. WO 01/70816, WO 02/066612, WO 02/066613, WO 02/066614, WO 02/066615, WO 02/29075, and WO 2005/108617. An example of a non-steroidal ecdysone agonist-regulated system is the RheoSwitch^{®} Mammalian Inducible Expression System (New England Biolabs, Ipswich, MA).

To introduce the therapeutic polynucleotide into the cells, a vector, comprising the chosen promoter and the polynucleotide encoding the lethal polypeptide can be used. The vector may be, for example, a plasmid vector, a single-or double-stranded phage vector, or a single-or double-stranded RNA or DNA viral vector. Such vectors may be introduced into cells by well-known techniques for introducing DNA and RNA into cells. Viral vectors may be replication competent or replication defective. In the latter case, viral propagation generally will occur only in complementing host cells. As used herein, the term "host cell" or "host" is used to mean a cell of the present invention that is harboring one or more therapeutic polynucleotides.

Thus, at a minimum, the vectors must include a promoter from a disease marker gene and a polynucleotide encoding a lethal polypeptide. Other components of the vector may include, but are not limited to, selectable markers, chromatin modification domains, additional promoters driving expression of other polypeptides that may also be present on the vector, genomic integration sites, recombination sites, and molecular insertion pivots. The vectors may comprise any number of these additional elements such that the construct can be tailored to the specific goals of the treatment methods desired.

In one embodiment of the present invention, the vectors that are introduced into the cells further comprise a "selectable marker gene" which, when expressed, indicates that the therapeutic construct of the present invention has been integrated into the genome of the host cell. In this manner, the selector gene can be a positive marker for the genome integration. While not critical to the methods of the present invention, the presence of a selectable marker gene allows the practitioner to select for a population of live cells where the vector construct has been integrated into the genome of the cells. Thus, certain embodiments of the present invention comprise selecting cells where the vector has successfully been integrated. As used herein, the term "select" or variations thereof, when used in conjunction with cells, is intended to mean standard, well-known methods for choosing cells with a specific genetic make-up or phenotype. Typical methods include, but are not limited to culturing cells in the presence of antibiotics, such as G418, neomycin and ampicillin. Other examples of selectable marker genes include, but are not limited to, genes that confer resistance to dihydrofolate reductase, hygromycin, or mycophenolic acid. Other methods of selection include, but are not limited to, a selectable marker gene that allows for the use of thymidine kinase, hypoxanthine-guanine phosphoribosyltransferase and adenine phosphoribosyltransferase as selection agents. Cells comprising a vector construct comprising an antibiotic resistance gene or genes would then be capable of tolerating the antibiotic in culture. Likewise, cells not comprising a vector construct comprising an antibiotic resistance gene or genes would not be capable of tolerating the antibiotic in culture.

As used herein, a "chromatin modification domain" (CMD) refers to nucleotide sequences that interact with a variety of proteins associated with maintaining and/or altering chromatin structure, such as, but not limited to, DNA insulators. *See* Ciavatta, D., et al., Proc. Nat'l Acad. Sci. U.S.A., 103(26):9958-9963 (2006). Examples of CMDs include, but are not limited to, the chicken β-globulin insulator and the chicken hypersensitive site 4 (cHS4). The use of different CMD sequences between one or more gene programs, for example, can facilitate the use of the differential CMD DNA sequences as "mini homology arms" in combination with various microorganism or *in vitro* recombineering technologies to "swap" gene programs between existing multigenic and monogenic shuttle vectors. Other examples of chromatin modification domains are known in the art or can be readily identified.

Particular vectors for use with the present invention are expression vectors that code for proteins or portions thereof. Generally, such vectors comprise *cis-*acting control regions effective for expression in a host operatively linked to the polynucleotide to be expressed. Appropriate *trans-acting* factors are supplied by the host, supplied by a complementing vector or supplied by the vector itself upon introduction into the host.

A great variety of expression vectors can be used to express proteins. Such vectors include chromosomal, episomal and virus-derived vectors, *e.g.,* vectors derived from bacterial plasmids, from bacteriophage, from yeast episomes, from yeast chromosomal elements, from viruses such as adeno-associated viruses, lentiviruses, baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. All may be used for expression in accordance with this aspect of the present invention. Generally, any vector suitable to maintain, propagate or express polynucleotides or proteins in a host may be used for expression in this regard.

The DNA sequence in the expression vector is operatively linked to appropriate expression control sequence(s) including, for instance, a promoter to direct mRNA transcription. Representatives of additional promoters include, but are not limited to, constitutive promoters and tissue specific or inducible promoters. Examples of constitutive eukaryotic promoters include, but are not limited to, the promoter of the mouse metallothionein I gene (Hamer et al., J. Mol. Appl. Gen. 1:273-288 (1982)); the TK promoter of Herpes virus (McKnight, Cell 31:355-365 (1982)); the SV40 early promoter (Benoist, et al., Nature (London) 290:304-310 (1981)); and the vaccinia virus promoter. Additional examples of the promoters that could be used to drive expression of a protein include, but are not limited to, tissue-specific promoters and other endogenous promoters for specific proteins, such as the albumin promoter (hepatocytes), a proinsulin promoter (pancreatic beta cells) and the like. In general, expression constructs will contain sites for transcription, initiation and termination and, in the transcribed region, a ribosome binding site for translation. The coding portion of the mature transcripts expressed by the constructs may include a translation initiating AUG at the beginning and a termination codon (UAA, UGA or UAG) appropriately positioned at the end of the polypeptide to be translated.

In addition, the constructs may contain control regions that regulate, as well as engender expression. Generally, such regions will operate by controlling transcription, such as repressor binding sites and enhancers, among others.

The vector containing an appropriate nucleotide sequence, as well as an appropriate promoter, and other appropriate control sequences, may be introduced into a cell of the present invention using a variety of well-known techniques that are suitable for the expression of a desired polypeptide.

Examples of eukaryotic vectors include, but are not limited to, pW-LNEO, pSV2CAT, pOG44, pXT1 and pSG available from Stratagene; pSVK3, pBPV, pMSG and pSVL available from Amersham Pharmacia Biotech; and pCMVDsRed2-express, pIRES2-DsRed2, pDsRed2-Mito, pCMV-EGFP available from Clontech. Many other vectors are well-known and commercially available.

Particularly useful vectors, which comprise molecular insertion pivots for rapid insertion and removal of elements of gene programs, are described in United States Published Patent Application No. 2004/0185556, United States Patent Application No. 11/233,246 and International Published Application Nos. WO 2005/040336 and WO 2005/116231. An example of such vectors is the UltraVector™ Production System (Intrexon Corp., Blacksburg, VA). As used herein, a "gene program" is a combination of genetic elements comprising a promoter (P), an expression sequence (E) and a 3' regulatory sequence (3), such that "PE3" as represented in the figures is a gene program. The elements within the gene program can be easily swapped between molecular pivots that flank each of the elements of the gene program. A molecular pivot, as used herein is defined as a polynucleotide comprising at least two non-variable rare or uncommon restriction sites arranged in a linear fashion. In one embodiment, the molecular pivot comprises at least three non-variable rare or uncommon restriction sites arranged in a linear fashion. Typically any one molecular pivot would not include a rare or uncommon restriction site of any other molecular pivot within the same gene program. Cognate sequences of greater than 6 nucleotides upon which a given restriction enzyme acts are referred to as "rare" restriction sites. There are, however, restriction sites of 6 bp that occur more infrequently than would be statistically predicted, and these sites and the endonucleases that cleave them are referred to as "uncommon" restriction sites. Examples of either rare or uncommon restriction enzymes include, but are not limited to, AsiS I, Pac I, Sbf I, Fse I, Asc I, Mlu I, SnaB I, Not I, Sal I, Swa I, Rsr II, BSiW I, Sfo I, Sgr AI, AflIII, Pvu I, Ngo MIV, Ase I, Flp I, Pme I, Sda I, Sgf I, Srf I, and Sse8781 I.

The vector may also comprise restriction sites for a second class of restriction enzymes called homing endonuclease (HE) enzymes. HE enzymes have large, asymmetric restriction sites (12-40 base pairs), and their restriction sites are infrequent in nature. For example, the HE known as I-SceI has an 18 bp restriction site (5TAGGGATAACAGGGTAAT3' (SEQ ID NO:1)), predicted to occur only once in every 7x10¹⁰ base pairs of random sequence. This rate of occurrence is equivalent to only one site in a genome that is 20 times the size of a mammalian genome. The rare nature of HE sites greatly increases the likelihood that a genetic engineer can cut a gene program without disrupting the integrity of the gene program if HE sites were included in appropriate locations in a cloning vector plasmid.

Selection of appropriate vectors and promoters for expression in a host cell is a well-known procedure, and the requisite techniques for vector construction and introduction into the host, as well as its expression in the host are routine skills in the art.

The introduction of the construct into the cells can be a transient transfection, stable transfection or can be a locus-specific insertion of the vector. Transient and stable transfection of the vectors into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods in Molecular Biology (1986); Keown et al., 1990, Methods Enzymol. 185: 527-37; Sambrook et al., 2001, Molecular Cloning, A Laboratory Manual, Third Edition, Cold Spring Harbor Laboratory Press, N.Y. These stable transfection methods result in random insertion of the vector into the genome of the cell. Further, the copy number and orientation of the vectors are also, generally speaking, random.

In one embodiment of the invention, the vector is inserted into a bio-neutral site in the genome. A bio-neutral site is a site in the genome where insertion of the construct interferes very little, if any, with the normal function of the cell. Bio-neutral sites may be analyzed using available bioinformatics. Many bio-neutral sites are known in the art, e.g., the ROSA-equivalent locus. Other bio-neutral sites may be identified using routine techniques well known in the art. Characterization of the genomic insertion site(s) is performed using methods known in the art. To control the location, copy number and/or orientation of the construct when introducing the vector into the cells, methods of locus-specific insertion may be used. Methods of locus-specific insertion are well-known in the art and include, but are not limited to homologous recombination and recombinase-mediated genome insertion. Of course, if locus-specific insertion methods are to be used in the methods of the present invention, the vectors may comprise elements that aid in this locus-specific insertion, such as, but not limited to, homologous recombination. For example, the vectors may comprise one, two, three, four or more genomic integration sites (GISs). As used herein, a "genomic integration site" is defined as a portion of the vector sequence which nucleotide sequence is identical or nearly identical to portions of the genome within the cells that allows for insertion of the vector in the genome. In particular, the vector may comprise two genomic insertion sites that flank at least the disease marker gene promoter and the polynucleotide encoding the lethal polypeptide. Of course, the GISs may flank additional elements, or even all elements present on the vector.

In another embodiment, locus-specific insertion may be carried out by recombinase-site specific gene insertion. Briefly, bacterial recombinase enzymes, such as, but not limited to, PhiC31 integrase can act on "pseudo" recombination sites within the human genome. These pseudo recombination sites can be targets for locus-specific insertion using the recombinases. Recombinase-site specific gene insertion is described in Thyagarajan, B. et al., Mol. Cell Biol. 21(12):3926-34 (2001). Other examples of recombinases and their respective sites that may be used for recombinase-site specific gene insertion include, but are not limited to, serine recombinases such as R4 and TP901-1.

Additional embodiments of the present invention include, but are not limited to, genotyping the cells. The term genotyping is used herein as it is in the art. Specifically, particular embodiments of the methods of the present invention provide for genotyping the cells either before or after inducing the killing of the diseased cells. Further, the methods contemplate genotyping the cells one or more times for quality assurance purposes. Genotyping may be carried in any way that provides genetic sequence information about all or a portion of the cells' genome. For example, genotyping can be carried out by isolating DNA and subsequent sequencing, or it may be carried out *via* PCR methods or restriction analysis or any combination thereof.

Genotyping of the cells of a subject may be carried out to determine the genomic profile of a subject. This information can be used to determine if a subject is predisposed to mutational events that would make the subject more susceptible to recurrence of the disease in subsequent generations of the non-diseased cells that were restored to the subject. A predisposition to mutational events in general may be identified by detection of alterations or mutations in the genes encoding DNA synthesis and repair genes or in other genes related to mutational events in the subject. A predisposition to a specific type of disease may be identified by detection of alterations or mutations in genes associated with that disease. Knowledge of the genotype of a subject may be used to design appropriate therapeutic polynucleotides for each subject. For example, if a subject is predisposed to a particular disease, a particular disease specific promoter or lethal polypeptide may be most suitable. If the subject is predisposed to recurrence of a disease, it would be preferable to not excise the therapeutic polynucleotide so that diseased cells may be purged *in vivo* at a later time if necessary. In another example, a subject's genotype may be used to determine of a particular insertion site in the genome is more or less suitable. Design of an individualized therapeutic polynucleotide based on a subject's genomic profile may be based on choices for each parameter of the polynucleotide as shown in Figure 1. A vector system in which parts are readily interchangeable, as described above, is ideally suited for assembling subject-specific therapeutic polynucleotides based on the genotype of the subject.

In addition, particular embodiments of the methods of the present invention comprise excision of the therapeutic polynucleotide. In general, the methods of the present invention will result in introduction of the therapeutic polynucleotides into all or most of the cells, regardless of the cells' disease state. Thus, it may be desirable to remove the therapeutic polynucleotide(s) from the non-diseased cells. To aid in its own excision the construct may therefore comprise additional elements such as recombinase sites. One example of a recombinase site includes, but is not limited to, the loxP site in the well-known cre-lox or the recombinase sites associated with the Int, IHF, Xis, Flp, Fis, Hin, Gin, Cin, Tn3 resolvase, ΦC31, TndX, XerC, and XerD recombinases. Other recombinase sites may be used, provided an appropriate recombinase enzyme can act upon the recombinase site. The construct may also contain genes coding for one or more recombinases. Expression of recombinases may be under the control of inducible promoters such that excision may be induced at the desired time.

The therapeutic polynucleotide may be excised from the surviving cells after the diseased cells have been destroyed and before the surviving cells are restored to the subject. Alternatively, surviving cells may be used to treat the subject without excising the therapeutic polynucleotide. In this situation, expression of the lethal polypeptide may be induced in vivo at any time after the cells are restored to the subject. This may be used if there is a recurrence of the disease in the subject. A recurrence may occur for any reason, including predisposition of the subject to mutational events that lead to recurrence of the disease in subsequent generations of the non-diseased cells that were restored to the subject. Recurrence may also occur due to incomplete purging of all of the diseased cells prior to restoration of the cells to the subject. The ability to destroy the diseased cells *in vivo (e.g.,* by having the lethal polypeptide under the control of an inducible promoter and providing the inducing agent to the subject) allows the subject to avoid an additional ex *vivo* transplantation cycle and the risks associated with the cycle *(e.g.,* the irradiation/chemotherapy required to eliminate the bone marrow prior to transplantation). This embodiment also allows the clinician to control the *in vivo* onset, level, and duration of the lethal polypeptide.

In a further embodiment, the therapeutic polynucleotide may comprise a chemo-resistance gene, e.g., the multidrug resistance gene mdrl. The chemo-resistance gene may be under the control of a constitutive *(e.g.,* CMV) or inducible *(e.g.,* RheoSwitch®) promoter. In this embodiment, if there is a recurrence of the disease in the subject, a clinician may apply a stronger dose of a chemotherapeutic agent to destroy diseased cells while the restored cells would be protected from the agent. By placing the chemo-resistance gene under an inducible promoter, the unnecessary expression of the chemo-resistance gene can be avoided, yet it will still be available in case of disease recurrence. If the restored cells themselves become diseased, they could still be destroyed by inducing expression of the lethal polypeptide as described above.

Still more embodiments contemplate analyzing and/or expanding the surviving cell population prior to using the cells to treat the subject. For example, the surviving cells may be genotyped and/or phenotyped, using any of the methods or protocols described or mentioned herein, prior to using the cells in the treatment of the subject.

The disclosure also describes kits that may be used in conjunction with methods the invention. Kits may comprise one or more containers, which may contain one or more components selected from the group consisting of one or more nucleic acid molecules, restriction enzymes and one or more cells comprising such nucleic acid molecules. Kits may further comprise one or more containers containing cell culture media suitable for culturing cells of the invention, one or more containers containing antibiotics suitable for use in culturing cells of the invention, one or more containers containing buffers, one or more containers containing transfection reagents, and/or one or more containers containing substrates for enzymatic reactions.

Kits may contain a wide variety of nucleic acid molecules that can be used with the invention. Examples of nucleic acid molecules that can be supplied in kits include those that contain promoters, sequences encoding lethal polypeptides, enhancers, repressors, selection markers, transcription signals, translation signals, primer hybridization sites (e.g., for sequencing or PCR), recombination sites, restriction sites and polylinkers, sites that suppress the termination of translation in the presence of a suppressor tRNA, suppressor tRNA coding sequences, sequences that encode domains and/or regions, origins of replication, telomeres, centromeres, and the like. Nucleic acid molecules of the invention may comprise any one or more of these features in addition to a transcriptional regulatory sequence as described above.

Kits may comprise containers containing one or more recombination proteins. Suitable recombination proteins include, but are not limited to, Cre, Int, IHF, Xis, Flp, Fis, Hin, Gin, Cin, Tn3 resolvase, ΦC31, TndX, XerC, and XerD. Other suitable recombination sites and proteins are those associated with the GATEWAY™ Cloning Technology available from Invitrogen Corp., Carlsbad, CA, and described in the product literature of the GATEWAY™ Cloning Technology.

In use, a nucleic acid molecule comprising one or more disease marker gene promoters (P) provided in a kit of the invention may be combined with an expression polynucleotide for a lethal polypeptide (E) and a 3' regulatory sequence (3) to prepare a PE3 gene program. The nucleic acid molecule comprising one or more 3' regulatory sequences may be provided, for example, with a molecular pivot on the 5' and 3' ends of the 3' regulatory sequence.

Kits can also be supplied with primers. These primers will generally be designed to anneal to molecules having specific nucleotide sequences. For example, these primers can be designed for use in PCR to amplify a particular nucleic acid molecule. Sequencing primers may also be supplied with the kit.

One or more buffers (e.g., one, two, three, four, five, eight, ten, fifteen) may be supplied in kits These buffers may be supplied at working concentrations or may be supplied in concentrated form and then diluted to the working concentrations. These buffers will often contain salt, metal ions, co-factors, metal ion chelating agents, etc. for the enhancement of activities or the stabilization of either the buffer itself or molecules in the buffer. Further, these buffers may be supplied in dried or aqueous forms. When buffers are supplied in a dried form, they will generally be dissolved in water prior to use.

Kits may contain virtually any combination of the components set out above or described elsewhere herein. As one skilled in the art would recognize, the components supplied with kits of the invention will vary with the intended use for the kits. Thus, kits may be designed to perform various functions set out in this application and the components of such kits will vary accordingly.

The disclosure also relates to instructions for performing one or more methods of the invention. Such instructions can instruct a user of conditions suitable for performing methods of the invention. Instructions can be in a tangible form, for example, written instructions (e.g., typed on paper), or can be in an intangible form, for example, accessible *via* a computer disk or over the internet.

It will be recognized that a full text of instructions for performing a method of the invention or, where the instructions are included with a kit, for using the kit, need not be provided. One example of a situation in which a kit of the invention, for example, would not contain such full length instructions is where the provided directions inform a user of the kits where to obtain instructions for practicing methods for which the kit can be used. Thus, instructions for performing methods of the invention can be obtained from internet web pages, separately sold or distributed manuals or other product literature, etc. For example kits may direct a kit user to one or more locations where instructions not directly packaged and/or distributed with the kits can be found. Such instructions can be in any form including, but not limited to, electronic or printed forms.

The following examples are illustrative, but not limiting, of the methods of the present invention. Other suitable modifications and adaptations of the variety of conditions and parameters normally encountered in medical treatment and gene expression systems and which are obvious to those skilled in the art are within the scope of the invention.

### Examples

### Example 1

CD34⁺ cells are obtained and isolated from the blood of a patient with myeloid leukemia. The isolated cells are treated *ex vivo* with a therapeutic polynucleotide construct of the present invention comprising a neomycin (neo) selection gene under the control of the constitutive, ubiquitous cytomegalovirus (CMV) promoter, and the inducible trans-activator rTTA under the control of the TERT promoter. A diphtheria (DTA) toxin coding sequence is under the control of the TetO inducible promoter. Cell transformation is then performed under conditions that promote random integration of the therapeutic construct into the genome. Stable transformants are selected by the addition of G418. Surviving colonies are then treated with tetracycline or doxycycline to activate expression of the DTA lethal gene product in those cells wherein the TERT promoter is driving expression of the rTTA protein product. Addition of tetracycline or doxycycline and expression of rTTA leads to the killing of diseased cells wherein the TERT promoter is active.

The vector shown in FIGURE 6 is one example of a vector design that may be useful for the methods of the present example. Characterization of the genomic insertion site(s) is performed using methods known in the art. In one embodiment, the cells where the constructs have been inserted into a bio-neutral site are grown and expanded under conditions that provide selective pressure on cells comprising the constructs.

These expanded colonies are phenotyped to indicate the presence or absence of disease marker genes. Phenotype may also be assessed to determine the ability of the cells to maintain the plasticity of a progenitor cell as assessed by known progenitor biomarkers, including but not limited to CD34⁺ biomarkers. The cell populations that pass this final phenotypic analysis will then be transplanted into the donor patient. Prior to transplantation, the patients may have their blood cells/bone marrow ablated via chemical or radioactive methodologies.

### Example 2

Peripheral blood cells are obtained and isolated from the blood of a patient with cancer of blood cell origin. The isolated cells are treated *ex vivo* with a therapeutic polynucleotide construct of the present invention comprising a neomycin (neo) selection gene under the control of the aldehyde dehydrogenase promoter, and the inducible trans-activator rTTA under the control of the TERT promoter. A diphtheria (DTA) toxin coding sequence is under the control of the TetO inducible promoter, and a CMV constitutive promoter controls expression of the RheoCept^{®} trans-activator. In turn, the RheoSwitch^{®} inducible promoter controls expression of the Cre recombinase. LoxP sites flank each of the gene programs of the construct at the 5' and 3' ends of the construct. Cell transformation is performed under conditions that promote random integration of the heterologous DNA into the genome. G418 is added to the cell culture to select for stable transformants that display a progenitor cell phenotype, because the aldehyde dehydrogenase promoter is expressed at high levels in progenitor cells comprising the construct. Surviving colonies are then treated with tetracycline or doxycyline to activate expression of the DTA lethal gene product in cells wherein the TERT promoter is driving expression of the rTTA protein product.

The vector shown in FIGURE 7 is one example of a vector design that may be useful for the methods of the present example. Characterization of the genomic insertion site(s) is performed using methods known in the art. In one embodiment, the cells where the constructs have been inserted into a bio-neutral site are grown and expanded under conditions that provide selective pressure on cells comprising the constructs.

These expanded colonies are phenotyped to indicate the presence or absence of disease marker genes. Phenotype may also be assessed to determine the cells ability to maintain the plasticity of a progenitor cell as assessed by known progenitor biomarkers, including but not limited to CD34⁺ and aldehyde dehydrogenase. The cell populations that pass this final phenotypic analysis will then be transplanted into the donor patient. Prior to transplantation, the patients may have their blood cells/bone marrow ablated *via* chemical or radioactive methodologies. Excision of the vector may be induced at any time by exposing the cells to an ecdysone receptor agonist to induce expression of the Cre recombinase.

### Example 3

To prevent metastasis and reduce circulating cancer cells, e.g., breast cancer cells, in a post-surgical cancer patient, peripheral blood cells are obtained and isolated from a patient that has or had breast cancer. These isolated cells are treated *ex vivo* with a therapeutic construct comprising the cytodine deaminase (CDA) gene under control of the ubiquitous, constitutively expressed phospho-glycerate kinase (PGK) promoter, and a portion of DNA that is homologous to the bio-neutral ROSA-equivalent locus. In the construct, the neo selection gene is under the control of the aldehyde dehydrogenase promoter, and the inducible trans-activator rTTA is under the control of the TERT promoter. In addition, the herpes simplex virus (HSV) thymidine kinase (TK) coding sequence is under the control of the TetO inducible promoter, and an additional region of DNA homologous to the bio-neutral ROSA-equivalent locus that lies 3' of the first DNA region. Also, the diphtheria (DTA) toxin gene is under the control of the constitutively expressed CMV promoter. Cell transformation is performed under conditions that promote locus-specific insertion via homologous recombination. Stable transformants displaying a progenitor cell phenotype are selected for by the addition of G418 because the aldehyde dehydrogenase promoter is expressed at high levels in progenitor cells.

The vector shown in FIGURE 8 is one example of a vector design that may be useful for the methods of the present example. Specifically, the construct is inserted into the genome at the bio-neutral ROSA-equivalent locus. Loss of the DTA selector of the construct indicates that homologous recombination was successful. Treatment with 5-fluorocytosine serves as a negative selector for loss of the CDA selector gene. Characterization of the genomic insertion site is performed using methods known in the art.

Colonies wherein the portions of the construct internal to the homologous recombination regions that have integrated into the genome at the bio-neutral ROSA-equivalent locus are selected and expanded. These expanded cells are then treated with tetracycline or doxycycline in addition to gancyclovir. Treatment with tetracycline or doxycycline will lead to activation of the TetO promoter in cells expressing the disease marker gene of choice, which in turn will activate expression of the TK gene product. Gancyclovir treatment selectively kills cells expressing thymidine kinase at high expression levels.

These expanded colonies are phenotyped to indicate the presence or absence of disease marker genes. Phenotype may also be assessed to determine the cells ability to maintain the plasticity of a progenitor cell as assessed by known progenitor biomarkers, including but not limited to CD34⁺ and aldehyde dehydrogenase. The cell populations that pass this final phenotypic analysis will then be transplanted into the donor patient. Prior to transplantation, the patient may have their blood cells/bone marrow ablated *via* chemical or radioactive methodologies.

### Example 4

Peripheral blood cells are obtained and isolated from the blood of a patient with cancer of blood cell origin. The isolated cells are treated *ex vivo* with a therapeutic polynucleotide construct of the present invention comprising a neomycin (neo) selection gene under the control of the aldehyde dehydrogenase promoter, and the diphtheria (DTA) toxin coding sequence under the control of the TERT promoter. Cell transformation is performed under conditions that promote random integration of the heterologous DNA into the genome. G418 is added to the cell culture to select for stable transformants that display a progenitor cell phenotype, because the aldehyde dehydrogenase promoter is expressed at high levels in progenitor cells comprising the construct.

Characterization of the genomic insertion site(s) is performed using methods known in the art. In one embodiment, the cells where the constructs have been inserted into a bio-neutral site are grown and expanded under conditions that provide selective pressure on cells comprising the constructs.

These expanded colonies are phenotyped to indicate the presence or absence of disease marker genes. Phenotype may also be assessed to determine the ability of the cells to maintain the plasticity of a progenitor cell as assessed by known progenitor biomarkers, including but not limited to CD34⁺ and aldehyde dehydrogenase. The cell populations that pass this final phenotypic analysis will then be transplanted into the donor patient without excision of the therapeutic polynucleotide. Prior to transplantation, the patients may have their blood cells/bone marrow ablated *via* chemical or radioactive methodologies.

Upon recurrence of the blood cell cancer in the patient, expression of DTA from the TERT promoter will be activated in the transplanted cells and these cells will be destroyed.

### Example 5

Peripheral blood cells are obtained and isolated from the blood of a patient with cancer of blood cell origin. The isolated cells are treated *ex vivo* with a therapeutic polynucleotide construct of the present invention comprising a neomycin (neo) selection gene under the control of the aldehyde dehydrogenase promoter, and the inducible trans-activator RheoCept^{®} trans-activator under the control of the TERT promoter. In turn, the RheoSwitch^{®} inducible promoter controls expression of DTA. Cell transformation is performed under conditions that promote random integration of the heterologous DNA into the genome. G418 is added to the cell culture to select for stable transformants that display a progenitor cell phenotype, because the aldehyde dehydrogenase promoter is expressed at high levels in progenitor cells comprising the construct. Surviving colonies are then treated with a RheoCept^{®} agonist to activate expression of the DTA lethal gene product in cells wherein the TERT promoter is driving expression of the RheoCept^{®} protein product.

Characterization of the genomic insertion site(s) is performed using methods known in the art. In one embodiment, the cells where the constructs have been inserted into a bio-neutral site are grown and expanded under conditions that provide selective pressure on cells comprising the constructs.

These expanded colonies are phenotyped to indicate the presence or absence of disease marker genes. Phenotype may also be assessed to determine the cells' ability to maintain the plasticity of a progenitor cell as assessed by known progenitor biomarkers, including but not limited to CD34⁺ and aldehyde dehydrogenase. The cell populations that pass this final phenotypic analysis will then be transplanted into the donor patient without excision of the therapeutic polynucleotide. Prior to transplantation, the patients may have their blood cells/bone marrow ablated *via* chemical or radioactive methodologies.

Upon recurrence of the blood cell cancer in the patient, a RheoCept^{®} agonist is administered to the patient, thereby inducing expression of the DTA gene product in transplanted cells or their progeny wherein the TERT promoter is driving expression of the RheoCept^{®} protein product.

## Claims

1. An *ex vivo* method of obtaining live, non-diseased cells said cells being suitable for treating a disease by destroying diseased cells in a subject, wherein said cells are obtained by a method comprising
(a) determining the activity of at least one disease marker gene within a population of cells obtained from said subject;
(b) introducing into said cells a polynucleotide that encodes a selectable marker and a polypeptide that is itself lethal to said cells, wherein the expression of said lethal polypeptide is directly or indirectly controlled by the promoter of said at least one disease marker gene;
(c) exposing said cells to selection conditions to obtain cells comprising said polynucleotide;
(d) treating said cells with conditions to induce expression of said lethal polypeptide, wherein said expression of said lethal polypeptide kills said cells expressing said at least one disease marker gene; and
(e) separating said killed cells from the remaining live, non-diseased cells, wherein said live cells do not express said lethal polypeptide to an extent sufficient to kill said non-diseased cells.

2. The method of claim 1, wherein said promoter is operably linked to said polynucleotide encoding said lethal polypeptide.

3. The method of claim 1, wherein said cells are selected from the group consisting of hematopoietic stem cells, liver stem cells, mammary stem cells, pancreatic stem cells, and neuronal stem cells.

4. The method of claim 3, wherein said cells are hematopoietic stem cells.

5. The method of claim 1, wherein said introducing said polynucleotide comprises transient transfection of said polynucleotide into said cells.

6. The method of claim 1, wherein said introducing said polynucleotide comprises stable transfection of said polynucleotide into said cells.

7. The method of claim 1, wherein said polynucleotide comprises at least two gene programs.

8. The method of claim 1, wherein said promoter of said disease marker gene is ligated between a first and a second molecular insertion pivot.

9. The method of claim 7, wherein said polynucleotide encoding said lethal polypeptide is ligated between a second and a third molecular insertion pivot.

10. The method of claim 8 or 9, wherein said molecular insertion pivots are comprised of three or four rare or uncommon restriction sites in a contiguous arrangement, said rare or uncommon restriction sites being selected from the group consisting of the restriction sites correlating to the AsiS I, Pac I, Sbf I, Fse I, Asc I, MIu I, SnaB I, Not I, Sal I, Swa I, Rsr II, BSiW I, Sfo I, Sgr AI, AfI III, Pvu I, Ngo MIV, Ase I, FIp I, Pme I, Sda I, Sgf I, Srf I and Sse878 I restriction enzymes.

11. The method of claim 7, wherein polynucleotide further comprises at least one chromatin modification domain.

12. The method of claim 1, wherein said introducing said polynucleotide comprises locus- specific insertion of said polynucleotide.

13. The method of claim 1, wherein said polynucleotide is contained in a vector.

14. The method of claim 1, wherein said vector is a viral vector.

## Patentansprüche

1. Ex-vivo-Verfahren zum Erhalten lebender, nicht erkrankter Zellen, wobei die Zellen für das Behandeln einer Krankheit geeignet sind, indem erkrankte Zellen in einem Individuum vernichtet werden, wobei die Zellen mit einem Verfahren erhalten werden, das Folgendes enthält:
(a) Bestimmen der Aktivität mindestens eines Krankheitsmarkergens in einer Population von Zellen, die von dem Individuum erhalten wurden;
(b) Einführen eines Polynukleotids, das einen selektierbaren Marker und ein Polypeptid kodiert, das an sich für die Zellen letal ist, in die Zellen, wobei die Expression des letalen Polypeptids direkt oder indirekt von dem Promotor des mindestens einen Krankheitsmarkergens kontrolliert wird;
(c) Aussetzen der Zellen gegenüber Selektionsbedingungen, um Zellen zu erhalten, die das Polynukleotid enthalten;
(d) Behandeln der Zellen mit Bedingungen, um die Expression des letalen Polypeptids zu induzieren, wobei die Expression des letalen Polypeptids die Zellen, die das mindestens eine Krankheitsmarkergen exprimieren, tötet; und
(e) Trennen der getöteten Zellen von den verbleibenden lebenden, nicht erkrankten Zellen, wobei die lebenden Zellen das letale Polypeptid nicht in ausreichendem Maß exprimieren, um die nicht erkrankten Zellen zu töten.

2. Verfahren nach Anspruch 1, wobei der Promotor mit dem Polynukleotid, welches das letale Polypeptid kodiert, operativ verknüpft ist.

3. Verfahren nach Anspruch 1, wobei die Zellen aus der Gruppe ausgewählt sind, die aus hämatopoietischen Stammzellen, Leberstammzellen, Mamma-Stammzellen, Bauchspeicheldrüsen-Stammzellen und neuronalen Stammzellen besteht.

4. Verfahren nach Anspruch 3, wobei es sich bei den Zellen um hämatopoietische Stammzellen handelt.

5. Verfahren nach Anspruch 1, wobei das Einführen des Polynukleotids eine transiente Transfektion des Polynukleotids in die Zellen umfasst.

6. Verfahren nach Anspruch 1, wobei das Einführen des Polynukleotids eine stabile Transfektion des Polynukleotids in die Zellen umfasst.

7. Verfahren nach Anspruch 1, wobei das Polynukleotid mindestens zwei Genprogramme enthält.

8. Verfahren nach Anspruch 1, wobei der Promotor des Krankheitsmarkergens zwischen einem ersten und einem zweiten molekularen Einführungs-Drehpunkt ligiert ist.

9. Verfahren nach Anspruch 7, wobei das Polynukleotid, welches das letale Polypeptid kodiert, zwischen einem zweiten und einem dritten molekularen Einführungs-Drehpunkt ligiert ist.

10. Verfahren nach Anspruch 8 oder 9, wobei die molekularen Einführungs-Drehpunkte aus drei oder vier seltenen oder ungewöhnlichen Restriktionsschnittstellen in einer kontigen Anordnung bestehen, wobei die seltenen oder ungewöhnlichen Restriktionsschnittstellen aus der Gruppe ausgewählt sind, die aus den Restriktionsschnittstellen in Korrelation mit den Restriktionsenzymen AsiSI, PacI, SbfI, FseI, AscI, MluI, SnaBI, NotI, SalI, SwaI, RsrII, BSiWI, SfoI, SgrAI, AflIII, PvuI, NgoMIV, AseI, FlpI, PmeI, SdaI, SgfI, SrfI und Sse878I besteht.

11. Verfahren nach Anspruch 7, wobei das Polynukleotid ferner mindestens eine Chromatinmodifizierungsdomäne enthält.

12. Verfahren nach Anspruch 1, wobei das Einführen des Polynukleotids ein locus-spezifisches Einführen des Polynukleotids umfasst.

13. Verfahren nach Anspruch 1, wobei das Polynukleotid in einem Vektor enthalten ist.

14. Verfahren nach Anspruch 1, wobei es sich bei dem Vektor um einen viralen Vektor handelt.

## Revendications

1. Procédé ex *vivo* d'obtention de cellules vivantes, non malades, lesdites cellules étant appropriées pour traiter une maladie en détruisant des cellules malades chez un sujet, dans lequel lesdites cellules sont obtenues par un procédé comprenant :
(a) la détermination de l'activité d'au moins un gène marqueur de maladie à l'intérieur d'une population de cellules obtenues dudit sujet ;
(b) l'introduction dans lesdites cellules d'un polynucléotide qui code un marqueur pouvant être sélectionné et d'un polypeptide qui est lui-même létal pour lesdites cellules, dans lequel l'expression dudit polypeptide létal est directement ou indirectement commandée par le promoteur dudit au moins un gène marqueur de maladie ;
(c) l'exposition desdites cellules à des conditions de sélection pour obtenir des cellules comprenant ledit polynucléotide ;
(d) le traitement desdites cellules avec des conditions pour induire l'expression dudit polypeptide létal, dans lequel ladite expression dudit polypeptide létal tue lesdites cellules exprimant ledit au moins un gène marqueur de maladie ; et
(e) la séparation desdites cellules tuées des cellules restantes vivantes, non malade, dans lequel lesdites cellules vivantes n'expriment pas ledit polypeptide létal dans une mesure suffisante pour tuer lesdites cellules non malades.

2. Procédé selon la revendication 1, dans lequel ledit promoteur est lié de manière opérationnelle audit polynucléotide codant ledit polypeptide létal.

3. Procédé selon la revendication 1, dans lequel lesdites cellules sont sélectionnées à partir du groupe constitué par des cellules souches hématopoïétiques, des cellules souches du foie, des cellules souches mammaires, des cellules souches pancréatiques et des cellules souches neuronales.

4. Procédé selon la revendication 3, dans lequel lesdites cellules sont des cellules souches hématopoïétiques.

5. Procédé selon la revendication 1, dans lequel ladite introduction dudit polynucléotide comprend la transfection transitoire dudit polynucléotide dans lesdites cellules.

6. Procédé selon la revendication 1, dans lequel ladite introduction dudit polynucléotide comprend la transfection stable dudit polynucléotide dans lesdites cellules.

7. Procédé selon la revendication 1, dans lequel ledit polynucléotide comprend au moins deux programmes génétiques.

8. Procédé selon la revendication 1, dans lequel ledit promoteur de gène marqueur de maladie est ligaturé entre un premier et un second pivot d'insertion moléculaire.

9. Procédé selon la revendication 7, dans lequel ledit polynucléotide codant ledit polypeptide létal est ligaturé entre une seconde et un troisième pivot d'insertion moléculaire.

10. Procédé selon la revendication 8 ou 9, dans lequel lesdits pivots d'insertion moléculaires sont composés de trois ou quatre sites de restriction rares ou peu communs dans un agencement contigu, lesdits sites de restriction rares ou peu communs étant sélectionnés à partir du groupe constitué par les sites de restriction se corrélant aux enzymes de restriction AsiS I, Pac I, Sbf I, Fse I, Asc I, MIu I, SnaB I, Not I, Sal I, Swa I, Rsr II, BSiW I, Sfo I, Sgr AI, AfI III, Pvu I, Ngo MIV, Ase I, FIp I, Pme I, Sda I, Sgf I, Srf I et Sse878 I.

11. Procédé selon la revendication 7, dans lequel ledit polynucléotide comprend en outre au moins un domaine de modification de chromatine.

12. Procédé selon la revendication 1, dans lequel ladite introduction dudit polynucléotide comprend une insertion spécifique au lieu géométrique dudit polynucléotide.

13. Procédé selon la revendication 1, dans lequel ledit polynucléotide est contenu dans un vecteur.

14. Procédé selon la revendication 1, dans lequel ledit vecteur est un vecteur viral.
